# EUROPEAN PATENT SPECIFICATION

(11) **EP 4 320 569 B1**
(45) Date of publication and mention of the grant of the patent: **23.07.2025**
(21) Application number: 22726203.7
(22) Date of filing: 06.04.2022
(51) Int. Cl.: G06Q 10/0833, G06Q 10/0832, G06Q 10/087, G06K 19/07, G06K 19/077, G16H 40/20

(54) **TRACEABLE MEDICAL VIALS**
VERFOLGBARE MEDIZINISCHE PHIOLEN
FLACONS MÉDICAUX TRAÇABLES

(30) Priority: 07.04.2021 US 202163171854 P
(43) Date of publication of application: 14.02.2024
(73) Proprietor: Genentech, Inc., South San Francisco, CA 94080-4990 (US)
(72) Inventor: COTTON, Gary Paul, South San Francisco, California 94080-4990 (US); DEROULHAC, Paul Gregoire, South San Francisco, California 94080-4990 (US); GUDURU, Sri Lakshmi, South San Francisco, California 94080-4990 (US)
(74) Representative: Mewburn Ellis LLP
(86) International application number: PCT/US2022/023694
(87) International publication number: WO 2022/216840

(56) References cited:
- EP-A1- 3 326 394
- WO-A1-2019/018837
- US-A1- 2019 037 362
- US-A1- 2019 120 929
- POPLI SAKSHI ET AL: "A Survey on Energy Efficient Narrowband Internet of Things (NBIoT): Architecture, Application and Challenges", IEEE ACCESS, vol. 7, 14 February 2019 (2019-02-14), pages 16739 - 16776, XP011709161, DOI: 10.1109/ACCESS.2018.2881533

## Description

### FIELD

Aspects described herein generally relate to trackable medical vials. More specifically, aspects described herein provide apparatuses, systems and methods for tracing and tracking medical vials throughout a medical distribution field, using hardware mounted to the medical vials.

### BACKGROUND

Medical manufacturers often sell their products to wholesalers, who in turn, resell the products to prescribing physicians. The flow of products after being sold to wholesalers may provide important information to manufacturers. This information may be valuable to manufacturers for improved product forecasting and inventory control. However, manufacturers' visibility to such product flow is often difficult. Data regarding the distribution of such products may be available from third parties, but such data is often expensive and time-intensive to obtain, and may not always provide complete information for all products. There is also a volume of expired product returned through the supply chain, and visibility does not exist to clearly and comprehensively identify the source of this issue so that steps can be taken to optimize inventory levels and mitigate costly expiration of product. Finally, there is an identified need among health care institutions to understand temperature variations at the individual unit level. A desire exists for visibility to a medical and pharmaceutical product's supply chain status and product viability (e.g., temperature control). At present, significant cost, design, and efficiency constraints stand in the way of such visibility.

US 2019/120929 A1 proposes a definable Blockchain in a Chip that can be inserted into product labels or packaging which can communicate via Bluetooth with a Smartphone or other Authenticated Radio Source. WO 2019/018837 A1 proposes a RFID label for use on a lid of a container, and a method of using the RFID label.

Therefore, improved apparatuses, systems and methods to address these and other shortcomings in the art are desired.

### SUMMARY

The invention is defined in the independent claims. Preferred embodiments are defined in the dependent claims.

The following presents a simplified summary of various aspects described herein. This summary is not an extensive overview, and is not intended to identify required or critical elements or to delineate the scope of the claims. The following summary merely presents some concepts in a simplified form as an introductory prelude to the more detailed description provided below.

To overcome limitations in the prior art described above, and to overcome other limitations that will be apparent upon reading and understanding the present specification, aspects described herein are directed towards tracking status information, such as location and temperature, of medical vials throughout at least a portion of their lifespan in a medical distribution field.

According to certain aspects of the present disclosure, a traceable vial is provided that includes a vial configured to hold contents therein, and a tracking assembly fitted to an exterior portion of the vial and housing electronic components configured to transmit a signal indicating a status of the vial.

The tracking assembly includes an antenna configured to transmit the signal and a battery configured to power the antenna. The tracking assembly includes a temperature sensor, and the antenna is configured to transmit a signal indicating a temperature reading by the temperature sensor. The tracking assembly is structured and designed to fit around a lower base portion of the vial. The tracking assembly may encircle a base of the vial.

In some examples, the electronic components housed in the tracking assembly may be configured to transmit the signal using a narrowband internet of things (NB-IOT) network. The electronic components housed in the tracking assembly may be configured to transmit the signal indicating at least one of: a vial identification, a location identification, a battery voltage, a coverage level, a radio frequency band, or a measured temperature. The electronic components housed in the tracking assembly may be configured to transmit the signal to a base transceiver station within a network, and lookup information associated with the nearest base transceiver station may indicate a location range of the traceable vial. Additionally, the tracking assembly may include a Wi-Fi antenna and may be configured to detect the presence of a local Wi-Fi signal which may be used to subsequently determine a location of the vial. The electronic components transmitting the signal to the base transceiver station may be configured to provide location mapping information including at least one of: a location name, a longitude, a latitude, or a location timestamp.

In some embodiments, the electronic components housed in the tracking assembly may be configured to transmit one of two messages in an alternating manner for a set update period. In such examples, a first message may include a device identification information, and a second message may include telemetry information associated with the traceable vial. The telemetry information associated with the traceable vial may include at least one of a temperature, or a battery voltage. The tracking assembly may be configured to deactivate upon receiving deactivation instructions from an administrator computing device or an end user computing device. In some examples, the vial may be composed of medical grade glass.

According to certain aspects of the present disclosure, a traceable vial system is provided that includes at least one traceable vial and a receiver. The at least one traceable vial may include a vial and a tracking assembly fitted to an exterior portion of the vial and housing electronic components configured to transmit a signal. The receiver may be configured to receive the signal from the at least one traceable vial and to detect one or more metrics of the at least one traceable vial based on the signal.

In some aspects, the receiver may be housed in a mobile user device. In such aspects, the mobile user device may execute an application that processes the signal received from the traceable vial. The mobile user device may transmit a second signal to an administrator computing device. The second signal may include location information identifying a location of the mobile user device when the signal was received from the traceable vial. In some examples, the signal may indicate a location information and a timestamp of when the signal was received.

These and additional aspects will be appreciated with the benefit of the disclosures discussed in further detail below.

### BRIEF DESCRIPTION OF THE DRAWINGS

A more complete understanding of aspects described herein and the advantages thereof may be acquired by referring to the following description in consideration of the accompanying drawings, in which like reference numbers indicate like features, and wherein:
FIGS. 1A and 1B depict an illustrative traceable medical vial, respectively showing a tracking assembly removed and a tracking assembly attached thereto in accordance with one or more illustrative aspects of the present disclosure.
FIG. 2 depicts a tracking assembly for a traceable medical vial in accordance with one or more illustrative aspects of the present disclosure.
FIG. 3 shows an operating environment for a traceable medical vial system in accordance with one or more illustrative aspects of the present disclosure.
FIG. 4 an analysis computing device in accordance with one or more aspects of the present disclosure.
FIG. 5 depicts a flowchart for operation of a traceable medical vial in accordance with one or more illustrative aspects of the present disclosure.

### DETAILED DESCRIPTION

In the following description of the various embodiments, reference is made to the accompanying drawings identified above and which form a part hereof, and in which is shown by way of illustration various embodiments in which aspects described herein may be practiced. It is to be understood that other embodiments may be utilized and structural and functional modifications may be made without departing from the scope described herein. Various aspects are capable of other embodiments and of being practiced or being carried out in various different ways.

As a general introduction to the subject matter described in more detail below, aspects described herein are directed towards constructing, initializing, tracking, and /or tracing medical vial apparatuses over a period of time in a medical distribution field, e.g., that includes wholesalers, pharmacies, hospitals, prescribing doctors, and/or patients. For example, apparatuses, systems methods of tracking medical vials in a medical distribution field are described herein. A traceable vial may include a vial configured to hold contents therein, and a tracking assembly fitted to an exterior portion of the vial and housing electronic components configured to transmit a signal indicating a status of the vial. As another example, a traceable vial system may include at least one traceable vial and a receiver. The at least one traceable vial may include a vial and a tracking assembly fitted to an exterior portion of the vial and housing electronic components configured to transmit a signal. The receiver may be configured to receive the signal from the at least one traceable vial and to detect one or more metrics of the at least one traceable vial based on the signal. By receiving the signal of one or more traceable medical vials in a medical distribution field, various data points associated with the movement of medical supplies throughout a medical distribution may become readily available, e.g., to medical manufacturers. Such information may enhance visibility into medical product flows, reduce the occurrence of unused medical products returns, improve product forecasting, enhance inventory control, increase patient safety, improve access for underserved populations, and the like. The traceable medical vial(s) as described herein provide a solution at the unit level to address medical supply chain and pharmaceutical product viability concerns which exist due to cost, design and efficiency constraints.

It is to be understood that the phraseology and terminology used herein are for the purpose of description and should not be regarded as limiting. Rather, the phrases and terms used herein are to be given their broadest interpretation and meaning. The use of "including" and "comprising" and variations thereof is meant to encompass the items listed thereafter and equivalents thereof as well as additional items and equivalents thereof. The use of the terms "connected," "coupled," "engaged" and similar terms, is meant to include both direct and indirect connecting, coupling, and engaging.

### Traceable Medical Vial Apparatuses

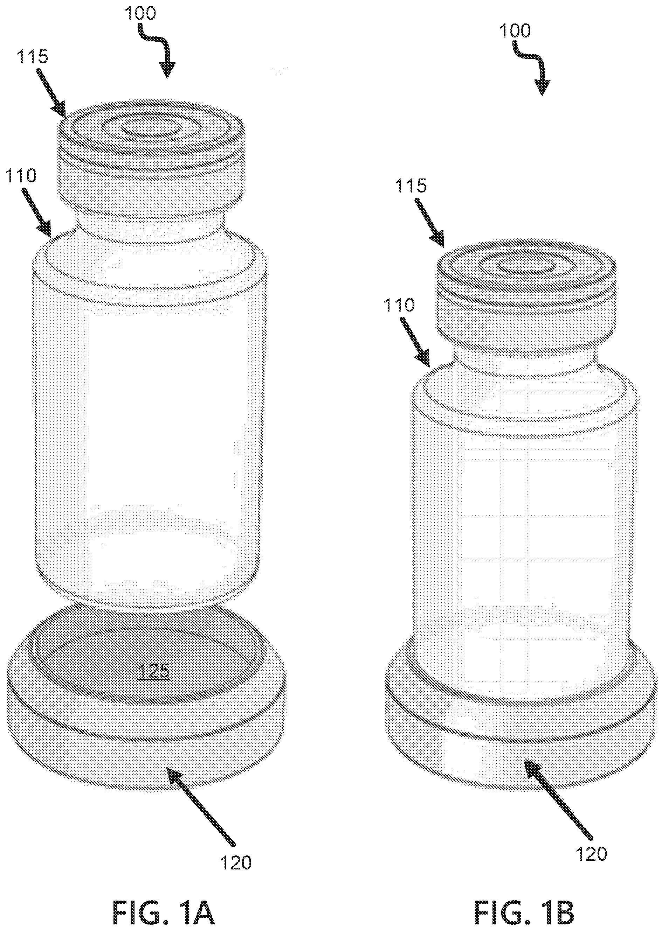

FIGS. 1A and 1B depict an illustrative traceable medical vial 100. As will be explained in greater detail below, such a medical vial may be used to track status information, e.g., location, temperature, content volume, battery power, etc., of the vial over a period of time. For example, a traceable medical vial such as the example traceable medical vial 100 may include a vial 110 configured to hold contents, e.g., a medical substance, therein. The vial 110 may be composed of a medical grade glass, such as borosilicate glass, soda lime glass, type II glass, and the like. In other examples, the vial 110 be me composed of plastics, such as polyethylene terephthalate, polyethylene, high density polyethylene (HDPE), low density polyethylene (LDPE), polyvinyl chloride (PVC), polypropylene, polystyrene, and the like. A closure device 115, such as an elastomeric closure, a screw top, a crown cap, a snap on lid, a friction fit closure, a temper-evident closure, a dispensing closure, a spray closure, a stopper, a bungs, and the like, may be provided with the vial 110. The closure device 115 may be designed and configured to retain contents within the vial, and to be removable in order to access the contents therein.

Included with the traceable medical vial 100, is a tracking assembly 120 housing, containing, or otherwise including, electronic components configured to transmit a signal indicating a status of the traceable medical vial 100, as will be discussed in more detail below. The tracking assembly 120 may be fitted to an exterior portion of the vial 110. For example, the tracking assembly 120 may be structured and designed to fit around a lower base portion of the traceable medical vial 100, and the tracking assembly 120 may encircle a base of the vial 110. As shown in FIG. 1A where the tracking assembly 120 is detached from the vial 110, the tracking assembly 120 includes a vial base compartment 125 sized and dimensioned to retain a lower base portion of the traceable medical vial 100 therein. Thus, when the tracking assembly 120 is fitted to the vial 110, as shown in FIG. 1B, the vial base compartment 125 may not be visible as the lower base portion of the vial 110 covers the vial base compartment 125. For example, the tracking assembly 120 may include various types of fittings for attachment to the vial 110, such as, but not limited to, pressure fits, snap fits, clearance fits, interference fits, mechanical fasteners, threaded fasteners, and the like

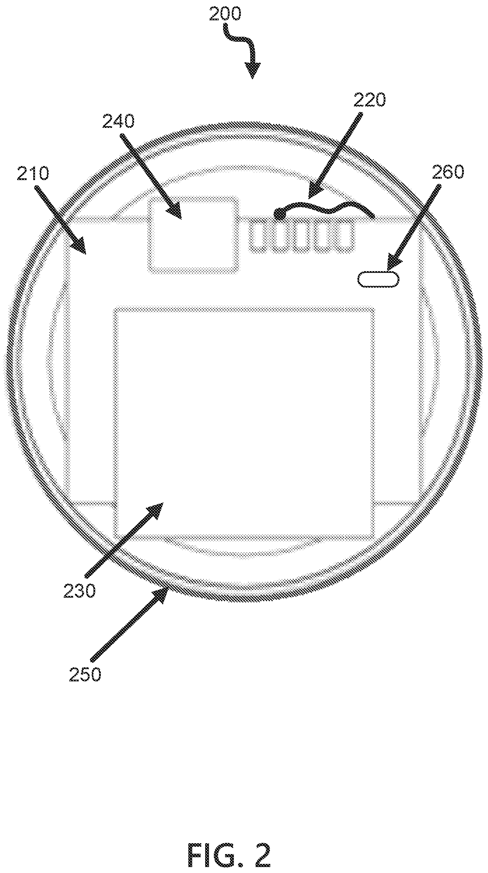

The tracking assembly 120 may include a number of electronic components suitable for transmitting a signal indicating on or more status of the traceable medical vial. Such electronic components may include temperature sensors, weight sensors, batteries, antennas, printed circuit boards, and the like, as will be described in more detail below. In some examples, the electronic components may be housed within a casing or other structure of the tracking assembly 120, such that the components are not visible, such as shown in the traceable medical vial 100 depicted in FIGS. 1A and 1B. In some examples, electronic components may be visible e.g., at a portion of the vial base compartment 125 and/or by removing a section of the external casing of the tracking assembly 120. As shown in FIG. 2, a tracking assembly 200 is depicted with a cover (not pictured) removed such that the electronic components housed therein are visible. The tracking assembly 120 of FIGS. 1A and 1B may include one or more of the same components as the tracking assembly 200 of FIG. 1.

Referring to FIG. 2, a tracking assembly 200 is shown in a view in which the electronic components are visible, e.g., by removal of a cover therefrom. In the view shown in FIG 2, casing 250 houses the electronic components within the tracking assembly 200. The casing 250 may be designed and sized so as to fit securely around a portion of the medical vial, such as a lower portion of the medical vial base. The tracking assembly 200 includes a printed circuit board 210 that supports and electrically connects a number of electronic components discussed herein, including an antenna 220 configured to transmit a signal and a battery 230 configured to power the antenna 220. The battery 230 may be selected from a variety of battery types sized appropriately to fit within casing 250 and to provide a suitable amount of power to the tracking assembly. For example, battery 230 may comprise a flexible lithium primary cell battery. Though not shown in FIG. 2, the tracking assembly 200 may include a switch configurable to turn battery power on and off to the electronic components therein.

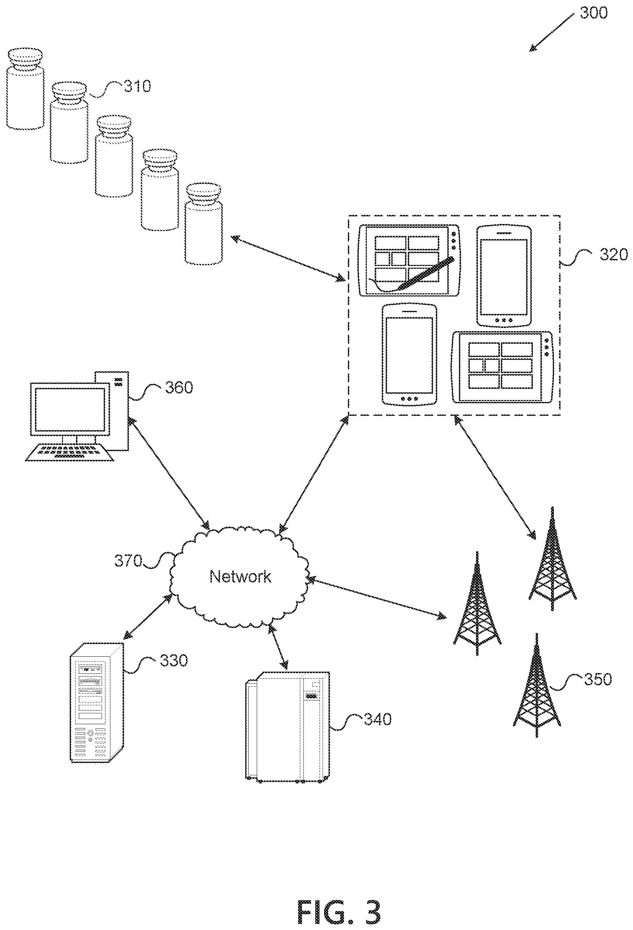

As partially shown in FIG. 2, antenna 220 may include a wire section that extends through at least a portion of the tracking assembly 200. In some instances, the antenna 220 may wrap extend over the casing 250 of the tracking assembly 200 and around an external portions of a vial. The antenna 220 may comprise a flexible PCB antenna (e.g., a Synzen SZP-K-0L02-A antenna) that is designed such that at least a portion of the antenna 220 can wrap around an outside portion of the vial. In some instances, the antenna 220 may be contained entirely within the casing 250 of the tracking assembly 200. The antenna 220 may be configured to transmit the signal using a narrowband internet of things (NB-IOT) network via an NB-IOT module 240. In some configurations, the tracking assembly 200 may include two antennas 220, e.g., in a base or lower portion of the casing 250. The two antennas 220 may include a Wi-Fi antenna and a NB-IoT antenna. The Wi-Fi antenna may be configured to detect and capture an identifier for the nearest Wi-Fi location. The NB-IoT antenna may be configured to communicate status and location information of the tracking assembly 200 as detected by the Wi-Fi antenna, e.g., through on or more transceiver stations 350 to the network 370 of Fig. 3, as discussed in more detail below. The tracking assembly 200 further includes a temperature sensor 260. The temperature sensor 260 may be electrically connected to the antenna 220 such that the antenna 220 is configured to transmit a signal indicating a temperature reading by the temperature sensor 260. Additional sensors or related devices may be included with the tracking assembly in accordance with various aspects of the present disclosure. For example, the tracking assembly may include a voltmeter that is configured to measure and transmit a battery voltage.

The antenna 220 may be configured to transmit a signal indicating at least one of: a vial identification, a location identification, a battery voltage, a coverage level, a radio frequency band, or a measured temperature, as will be described below in more detail. The antenna 220 may be configured to transmit a signal indicating additional types of status information relating to the traceable vial. For example, with regard to indicating a location identification, the antenna 220 may be configured to transmit a signal to a base transceiver station with a network. Lookup information associated with the base transceiver station may indicate a location range of the traceable vial. In some examples, the antenna 220 transmitting the signal to a base transceiver station may in turn provide location mapping information, such as a location name, a longitude, a latitude, a location timestamp, and the like. In some embodiments, the tracking assembly 200 may be configured to transmit one of two messages in an alternating manner for a set update period. For example, a first message may include a device identification information, and a second message may include telemetry information associated with the traceable vial, such as a temperate, a battery voltage, and the like. The tracking assembly 200 may include a switch or other similar component (not shown in FIG. 2) to activate the electronics therein at a specified time, as well as to deactivate the electronics, e.g., upon receiving deactivation instructions from an administrator computing device or an end user computing device. The FIG. 2 example is a hardware configuration, although some components may be wholly or partially implemented as software as well.

Modifications may be made to add, remove, combine, divide, etc. components of the traceable medical vial 100 and/or the tracking assembly 200 as desired. Additionally, the components may be implemented using basic electronic devices and components and medical container devices and component, and may be used to implement any of the other devices and components described herein. For example, the various additional or alternative electronic components herein may be implemented using electronic devices that provide similar functionality to those discussed herein.

### Medical Vial Tracing Systems

Discussion will now turn to use of systems implementing one or more traceable medical vial for tracking the medical vials throughout distribution is a medical network or field.

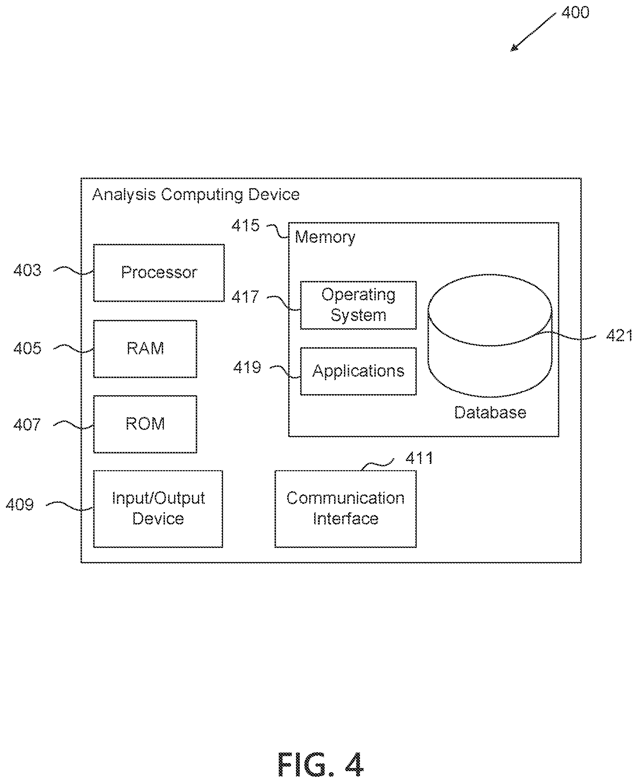

FIG. 3 shows an operating environment for a traceable medical vial system 300 in accordance with one or more illustrative aspects of the present disclosure. The traceable medical vial system 300 may include one or more traceable medical vial devices 310, one or more mobile computing devices 320, a processing server system 330, a recommendation server system 340, analysis computing device 360, and one or more transceiver station 350 in communication via a network 370. Analysis computing device 360 may be implemented, in whole or in part, using one or more computing systems described with respect to FIG. 4.

The one or more mobile computing devices 320 may receive status information signals from one or more traceable medical vial devices 310, and may transmit such status information signals and/or additional components of information via network 370 as described herein. The one or more mobile computing devices 320 may include a number of computing devices such as cellphones, smartphones, tablet computers, etc. The one or more mobile computing devices 320 may include one or more transceivers, digital signal processors, and additional circuitry and software for communicating via one or more network devices (e.g., base transceiver stations) in the wireless network.

An application associated with tracking may be installed on the mobile computing device 320, and the application may configure the mobile computing device 320 to transmit a beacon signal. The beacon signal may be transmitted based on a location of the mobile computing device 320. For example, the mobile computing device 320 may determine a location current, e.g., using a global navigation satellite system (GNSS) module on the mobile computing device 320, cell tower triangulation techniques via the one or more transceiver stations 350, and the like. The mobile computing device 320 may then transmit the beacon signal based on determining that it is in a vicinity of a first transceiver base station (e.g., within a threshold distance of the first transceiver base station). In another example, the mobile computing device 320 may transmit the beacon signal in response to receiving another beacon signal transmitted (e.g., periodically transmitted) by the first base receiver station. The beacon signal(s) may be near field communication (NFC) protocol signal(s), BLUETOOTH signal(s), mesh networking, ultra-wide band (UWB), crowd source networking, International Institution of Electrical and Electronic Engineers (IEEE) 802.11 WIFI signal(s), or signal(s) corresponding to any other wireless communication protocol. In some examples, where the one or more traceable medical vial devices 310 are equipped with an NB-IOT module, such as the NB-IOT module 240, the one or more traceable medical vial devices 310 may send a signal directly to the one or more transceiver stations 350 without relying on the mobile computing device 320. In such examples, this may be a primary mode of communication from the one or more traceable medical vial devices 310.

Processing server system 330 may obtain status information signals relating to one or more traceable medical vial devices 310, compute a variety of related parameters, and/or compile and group sets of traceable medical vial data as described in more detail herein. Recommendation server systems 340 may generate a variety of recommendations based on models and/or preference data as described herein. Analysis computing device 360 may receive data from processing server system 330 and recommendation server system 340, and may interactively display or otherwise provided processed information relating to the traceable medical vial system 300. The one or more traceable medical vial devices 310 may include a plurality of traceable medical vials, each having a vial configured to hold contents therein and a tracking assembly fitted to the vial and housing electronic components configured to transmit a signal indicating a status of the vial, e.g., as described in FIGS. 1 and 2. However, it should be noted that any of the one or more traceable medical vial devices 310, mobile computing devices 320, processing server system 330, recommendation server system 340, and/or analysis computing device 360 may perform some or all of any step of any process as described herein. The network 370 may include a local area network (LAN), a wide area network (WAN), a wireless telecommunications network, and/or any other communication network or combination thereof.

The one or more traceable medical vial devices 310 may be used to measure one or more properties of the vial, such as temperature, battery voltage, content volume, and the like. In some aspects, one or more mobile computing devices 320 may receive status information data from the one or more traceable medical vial devices 310, and may store such data in association with one or more vial parameters, e.g., to group data by a common vial contents or a common ship date from a manufacturer. In that regard, the traceable medical vial system 300 may include any number of traceable medical vial devices 310, of which various traceable medical vial devices 310 may be distributed throughout a medical distribution system at various different times.

As discussed herein, the data transferred to and from various devices in the traceable medical vial system 300 may include secure and proprietary data, such as medical sales documents, wholesaler sales data, medical data, and/or procedures for analyzing certain types of data. Therefore, it may be desirable to protect transmissions of such data using secure network protocols and encryption, and/or to protect the integrity of the data when stored on the various computing devices within the traceable medical vial system 300. For example, a file-based integration scheme or a service-based integration scheme may be used for transmitting data between the various computing devices. Data may be transmitted using various network communication protocols. Secure data transmission protocols and/or encryption can be used in file transfers to protect the integrity of the data, for example, File Transfer Protocol (FTP), Secure File Transfer Protocol (SFTP), and/or Pretty Good Privacy (PGP) encryption.

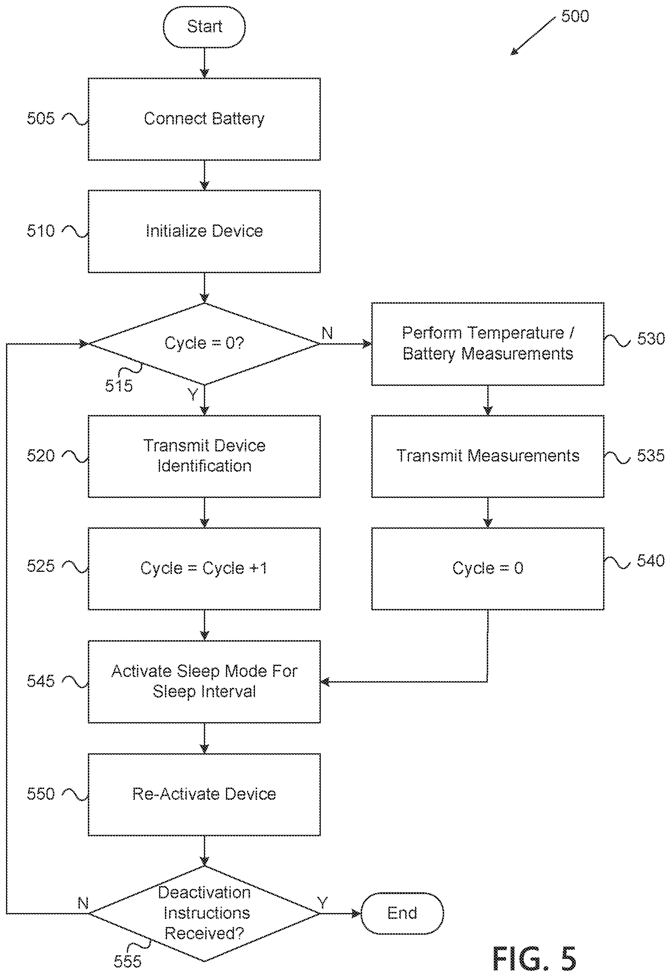

The traceable medical vial system 300 of FIG. 3 may be used in a low power wide area network (LPWAN). For example, the example system may be implemented using LPWAN protocol, and LPWAN protocol may be used to manage communication between the one or more traceable medical vial devices 310. In this regard, the traceable medical vial system 300 may also be used to implement any other type of LPWAN, such as SIGFOX, LTE-M, LORA, NB-IOT, and the like. For example, the one or more traceable medical vial devices 310 may be configured to transmit a signal using a narrowband internet of things (NB-IOT) network. The transmitted signal may include at least one of: a vial identification, a location identification, a battery voltage, a coverage level, a radio frequency band, or a measured temperature. In an example where the transmitted signal provides a location identification, the one or more traceable medical vial devices 310 may be configured to transmit the signal to a base transceiver station within a network, and lookup information associated with the nearest base transceiver station may indicate a location range of the traceable vial. The signal transmitted to the base transceiver station may thus provide location mapping information including at least one of: a location name, a longitude, a latitude, or a location timestamp. In some configurations, the one or more traceable medical vial devices 310 may be configured to detect the presence of a Wi-Fi signal and to capture an identifier associated with that Wi-Fi signal. The identifier associated with the Wi-Fi signal may be included in the information that is transmitted from the one or more traceable medical vial devices 310, e.g., in addition to latitude, longitude, temperature, battery condition, and the like. The identifier associated with the Wi-Fi signal may then be processed by the recommendation server system 340 to map the location of the one or more traceable medical vial devices 310. In another example, the one or more traceable medical vial devices 310 may be configured to transmit signals at a frequency that varies based on sensed movement of the vial device. For example, the time interval between consecutive transmitted signals from a particular vial device may be longer when the vial device is detected to be in a stationary location for a predetermined or extended period of time, as compared to when movement of the vial device has been detected. Such examples of varying signal transmission rates are described in further detail below with regard to FIG. 5.

In some embodiments, the one or more traceable medical vial devices 310 may be configured to transmit one of two messages in an alternating manner for a set update period. In such examples, a first message may include a device identification information, and a second message may include telemetry information associated with the traceable vial device. The telemetry information associated with the traceable vial device may include at least one of a temperature, or a battery voltage. The one or more traceable medical vial devices 310 may be configured to deactivate upon receiving deactivation instructions from an administrator computing device or an end user computing device.

In some embodiments, one or more web services may be implemented within the various computing devices. Web services may be accessed by authorized external devices and users to support input, extraction, and manipulation of data between the various computing devices in the traceable medical vial system 300. Web services built to support a personalized display system may be employed cross-domain and/or cross-platform, and may be built for enterprise use. Such web services may be developed in accordance with various web service standards, such as the Web Service Interoperability (WS-I) guidelines. Data may be transmitted using the Secure Sockets Layer (SSL) or Transport Layer Security (TLS) protocol to provide secure connections between the computing devices. Web services may be implemented using the WS-Security standard, which provides for secure SOAP messages using XML encryption. Specialized hardware may be used to provide secure web services. For example, secure network appliances may include built-in features such as hardware-accelerated SSL and HTTPS, WS-Security, and/or firewalls. Such specialized hardware may be installed and configured in the traceable medical vial system 300 in front of one or more computing devices such that any external devices can communicate directly with the specialized hardware.

It will be appreciated that the network connections shown are illustrative and other means of establishing a communications link between the computers may be used. The existence of any of various network protocols such as TCP/IP, Ethernet, FTP, HTTP and the like, and of various wireless communication technologies such as GSM, CDMA, WiFi, and WiMAX, is presumed, and the various computing devices described herein may be configured to communicate using any of these network protocols or technologies.

Turning now to FIG. 4, an analysis computing device 400, e.g., similar to analysis computing device 360 of FIG. 3, is shown. The analysis computing device 400 may include a processor 403 for controlling overall operation of the analysis computing device 400 and its associated components, including RAM 405, ROM 407, input/output device 409, communication interface 411, and/or memory 415. A data bus may interconnect processor(s) 403, RAM 405, ROM 407, memory 415, I/O device 409, and/or communication interface 411. Communication interface 411 may include one or more transceivers, digital signal processors, and/or additional circuitry and software for communicating via any network, wired or wireless, using any protocol including those described herein.

Input/output (I/O) device 409 may include a microphone, keypad, touch screen, and/or stylus through which a user of the analysis computing device 400 may provide input, and may also include one or more of a speaker for providing audio output and a video display device for providing textual, audiovisual, and/or graphical output. In some embodiments, the I/O devices 409 may include one or more sensors and/or one or more image capture devices. The image capture devices may be used to capture images of a subject. The sensors may be used to determine viscoelastic tissue parameters in images captured using one or more image capture devices. For example, I/O device 409 may include an ultrasound shear wave elastography apparatus. Software may be stored within memory 415 to provide instructions to processor 403 allowing the analysis computing device 400 to perform various actions. For example, memory 415 may store software used by the analysis computing device 400, such as an operating system 417, application programs 419, and/or an associated database 421. The various hardware memory units in memory 415 may include volatile and nonvolatile, removable and non-removable media implemented in any method or technology for storage of information such as computer-readable instructions, data structures, program modules, or other data. Memory 415 may include one or more physical persistent memory devices and/or one or more non-persistent memory devices. Memory 415 may include, but is not limited to, random access memory (RAM) 405, read only memory (ROM) 407, electronically erasable programmable read only memory (EEPROM), flash memory or other memory technology, CD-ROM, digital versatile disks (DVD) or other optical disk storage, magnetic cassettes, magnetic tape, magnetic disk storage or other magnetic storage devices, or any other medium that may be used to store the desired information and that may be accessed by processor 403.

Processor 403 may include a single central processing unit (CPU), which may be a single-core or multi-core processor (e.g., dual-core, quad-core, etc.), or may include multiple CPUs. Processor(s) 403 and associated components may allow the analysis computing device 400 to execute a series of computer-readable instructions to perform some or all of the processes described herein. Although not shown in FIG. 4, various elements within memory 415 or other components in analysis computing device 400, may include one or more caches, for example, CPU caches used by the processor 403, page caches used by the operating system 417, disk caches of a hard drive, and/or database caches used to cache content from database 421. For embodiments including a CPU cache, the CPU cache may be used by one or more processors 403 to reduce memory latency and access time. A processor 403 may retrieve data from or write data to the CPU cache rather than reading/writing to memory 415, which may improve the speed of these operations. In some examples, a database cache may be created in which certain data from a database 421 is cached in a separate smaller database in a memory separate from the database, such as in RAM 405 or on a separate computing device. For instance, in a multi-tiered application, a database cache on an application server may reduce data retrieval and data manipulation time by not needing to communicate over a network with a back-end database server. These types of caches and others may be included in various embodiments, and may provide potential advantages in certain implementations of document development systems, such as faster response times and less dependence on network conditions when transmitting and receiving data.

Although various components of analysis computing device 400 are described separately, functionality of the various components may be combined and/or performed by a single component and/or multiple computing devices in communication without departing from the scope of the present disclosure.

Methods of Operating Traceable Medical Vials and Obtaining Related Status Information

Discussion will now turn to use of traceable medical vials and collection of related status information therefrom.

FIG. 5 depicts a flowchart 500 for operation of a traceable medical vial in accordance with one or more illustrative aspects of the present disclosure. The steps shown in FIG. 5 may be performed by a computing device, such as the analysis computing device 360 or analysis computing device 400, or a traceable medical vial, in conjunction with a traceable medical vial device 310. The steps shown in FIG. 5 may be performed to operate one or more traceable medical vials and to collect relevant status information of the traceable medical vials as will be described herein.

At step 505, a battery may be connected to the traceable medical vial device. For example, a battery may be connected to a portion of a tracking assembly fitted to the traceable medical vial device, and the tracking assembly may house a number of electronic components which may be powered by the battery, such as an antenna, a temperature sensor, and the like. The battery connection step may include inserting a battery into a battery compartment of the traceable medical vial. As the traceable medical vial device may be operable for several months or even several years, the battery may be selected based on various known battery types capable of functioning for such durations of time. The step of connecting the battery to the traceable medical vial device may include assembling one or more electronic components in the traceable medical vial such that a battery-powered connection is formed. The step of connecting the battery to the traceable medical vial device may include assembling components of a traceable medical vial kit so as to assemble a traceable medical vial with the related electronic components connected to a battery. The step of connecting the battery to the traceable medical vial device may include removal of a pull tab from a traceable medical vial device that includes a battery assembled therein.

At step 510, the traceable medical vial device may be initialized. Initializing the traceable medical vial device may include starting, activating, or otherwise powering one or more electronic devices of the traceable medical vial device. In some instances, the traceable medical vial device may be configured to be initialized based on user activation of a switch on the traceable medical vial device. In some examples, the traceable medical vial device may be automatically initialized after connecting the battery at step 505. In some instances, the traceable medical vial device may be automatically initiating upon the occurrence of a certain event, e.g., at a certain preset time, after a certain time interval from which the battery was connected thereto, or upon leaving a manufacturer location.

At step 515, the traceable medical vial device may determine if a cycle count variable is set to zero. For example, the traceable medical vial device may be configured to initially have a cycle count variable set to zero.

If the cycle count variable is set to zero, then the traceable medical vial device may proceed to step 520 where the traceable medical vial device may transmit device identification information. Device identification information may include a stock-keeping unit (SKU), serial number, Universal Product Code (UPC), bar code, device name, and other similar device identifiers appropriate to indicate the device from which the signal was sent. Transmitting the device identification information may include one or more computing devices receiving a signal and passing the signal to one or more other computing device via a network, as discussed with respect to the system of FIG. 3.

For example, step 520 may include transmitting a signal with device identification information to a mobile device in a threshold vicinity of the traceable medial vial device. The mobile device may then send (e.g., transmit) a corresponding signal based on a location of the mobile device. For example, the mobile device may determine a current location, e.g., using a global navigation satellite system (GNSS) module on the mobile device, or using cell tower triangulation techniques. The transmitted signal may then include both location information (e.g., of the mobile device, which also indicates a location of the traceable medial vial), as well as the device identification information that was transmitted from the traceable medical vial device. The transmitted signal(s) may be near field communication (NFC) protocol signal(s), BLUETOOTH signal(s), International Institution of Electrical and Electronic Engineers (IEEE) 802.11 WIFI signal(s), or signal(s) corresponding to any other wireless communication protocol.

At step 525, the traceable medical vial device may add one to the cycle count variable (that was previously set to zero at determined at step 515). The cycle count variable may be updated at step 525 upon confirmation of the device identification information being transmitted at step 520 and/or upon confirmation that the transmitted device indentation information was received by a computing device. In some examples, the cycle count variable may be updated at step 525 after a preset time interval after transmitting the device identification information at step 520.

If the cycle count variable is not set to zero, then the traceable medical vial device may proceed to step 530 where the traceable medical vial device may perform one or more telemetry measurements, such as temperature measures or battery voltage measurements. As discussed herein, the traceable medical vial device may be configured with a number of electronic components suitable for performing a number of measurements relating to a status of the traceable medical vial device, such as temperature, battery voltage, vial content volume or weight, and the like. At step 530, the traceable medical vial device may causes these one or more electronic components perform suitable measurement.

At step 535, the traceable medical vial device may transmit the measurement information. Transmitting the measurement information at step 535 may be similar to transmitting the device identification information at step 520. For example, transmitting the measurement information may include one or more computing devices receiving a signal and passing the signal to one or more other computing devices via a network, as discussed with respect to the system of FIG. 3. For example, step 535 may include transmitting a signal with measurement information to a mobile device in a threshold vicinity of the traceable medial vial device. The mobile device may then send (e.g., transmit) a corresponding signal based on a location of the mobile device. For example, the mobile device may determine a current location, e.g., using a global navigation satellite system (GNSS) module on the mobile device, or using cell tower triangulation techniques. The transmitted signal may then include both location information (e.g., of the mobile device, which also indicates a location of the traceable medial vial), as well as the measurement information that was transmitted from the traceable medical vial device. The transmitted signal(s) may be near field communication (NFC) protocol signal(s), BLUETOOTH signal(s), International Institution of Electrical and Electronic Engineers (IEEE) 802.11 WIFI signal(s), or signal(s) corresponding to any other wireless communication protocol.

At step 540, the traceable medical vial device may reset the cycle count variable to zero. The cycle count variable may be updated at step 540 upon confirmation of the measurement information being transmitted at step 535 and/or upon confirmation that the transmitted measurement information was received by a computing device. In some examples, the cycle count variable may be updated at step 540 after a preset time interval after transmitting the measurement information at step 535. In this manner, the traceable medical vial device may send one of two alternating messages. As compared to sending both device identification information and device measurement information at the same time, the methods discussed herein of transmitting one of two alternating message effectively cuts the required transmission rate in half, thus also halving device power consumption and prolonging the battery life.

At step 545, the traceable medical vial device may activate or initiate a sleep mode for a sleep interval. By switching to a sleep mode, the traceable medical vial device may conserve battery power for certain intervals, e.g., soon after the previous transmission of status information, in which a change in status information is less likely. The sleep mode may entail that the traceable medical vial device does not perform operations, such that battery power used during the sleep interval is zero, or close to zero. In some instance, activation of the sleep mode at step 545 may occur upon updating the cycle count variable at step 525 or at step 540. In some instances, the sleep interval may be variable, e.g., based on an age of the traceable medical vial device, based on a level of battery power remaining, based on an indication that the contents of the traceable medical vial device may be close to expiration, and the like.

After the sleep interval, at step 550, the traceable medical vial device may be re-activated. For example, the traceable medical vial device may be automatically re-activated after the sleep interval time period has elapsed. As another example, the traceable medical vial device may be re-activated upon the occurrence of events, similar to the events described for initializing the device at step 510. For example, the traceable medical vial device may be configured to be re-activated based on user activation of a switch on the traceable medical vial device. In some examples, the traceable medical vial device may be automatically reactivated after the sleep interval period has elapsed and/or after a preset time from which a signal was previously transmitted from the traceable medical vial device. In some instances, the traceable medical vial device may be automatically re-activated upon the occurrence of a certain event, e.g., at a certain preset time, after a certain time interval from which the sleep mode was activated, and the like.

At step 555, the traceable medical vial device may determine if deactivation instructions have been received. In that regard, deactivation instruction may be received from a computing device, e.g., analysis computing device of FIG 4, at any instance during operation of the traceable medial vial device. Deactivation instructions may be received from an administrator computing device or an end user computing device. Deactivation instructions may include instructions to permanently deactivate the traceable medical vial device. In some instances, deactivation instructions may be automatically transmitted to the traceable medical vial device upon the occurrence of certain instances, such as based on a preset time period elapsing since the traceable medical device was first initialized, based on a last determined location of the traceable medical vial container, based on current battery power remaining reaching a certain threshold, and the like. In some examples, deactivation instructions may be received from one or more external computing devices such as analysis computing device. In some examples, deactivation instructions may be coded into certain components of the traceable medical vial device, but may not be received by a processor of the device until the occurrence of one or more preset events, as described herein. Deactivation instructions may be received at an interval in which an indication is made that the medical vial has been transferred to a patient or has been sold to an end-user. For example, this indication may be made based on identifying a device location outside of a pharmacy or hospital. As another example, the traceable medical vial device may include a deactivation switch to be engaged by a doctor, pharmacist or patient prior to the content of the vial being used.

If deactivation instructions have been received, the traceable medical vial device may be deactivated and the process flow may end. If deactivation instructions have not yet been received, however, the process may return to step 515, such that steps 515 through 555 may be repeated until deactivation instructions have been received.

In some examples, after initializing the device at step 510, the traceable medical vial device may toggle a locator device mode on and off. If the traceable medical vial device is set to a locator device mode, the traceable medical vial device may then proceed to transmit locator data, idle for an idle time interval (e.g., on the order of several seconds), repeat transmitting locator data and idling for the idle time until a locator mode time interval is up. When the locator mode time interval is up, the traceable medical vial device may return to step 515, such that steps 515 through 555 may be repeated. The locator mode may operate by transmitting locator data to any suitable mobile device, e.g., within an area of the traceable medical vial device, that is configured to communicate with a locator system, such as a Tile^{®} tracker system.

All step depicted in FIG. 5 may be repeated. The order of the various step may be modified, or the repetition of certain steps may occur in accordance with one or more aspects of the present disclosure. For example, the transmission of device identification information may repeat several times at step 520 before the traceable medical vial device proceeds to step 525 to update the cycle count variable.

Although the subject matter has been described in language specific to structural features and/or methodological acts, it is to be understood that the subject matter defined in the appended claims is not necessarily limited to the specific features or acts described above. Rather, the specific features and acts described above are described as example implementations of the following claims.

It should be understood that the analysis processes, method steps, and/or methods described herein may be performed in different orders and/or in alternative arrangements from those illustrated herein, without departing from the scope of this disclosure. Additionally or alternatively, one or more of the analysis processes, method steps, and/or methods described herein may be optional and/or omitted in some arrangements, without departing from the scope of this disclosure.

One or more aspects of the disclosure may be embodied in computer-usable data or computer-executable instructions, such as in one or more program modules, executed by one or more computers or other devices to perform the operations described herein. Program modules may include routines, programs, objects, components, data structures, and the like that perform particular tasks or implement particular abstract data types when executed by one or more processors in a computer or other data processing device. The computer-executable instructions may be stored as computer-readable instructions on a computer-readable medium such as a hard disk, optical disk, removable storage media, solid-state memory, RAM, and the like. The functionality of the program modules may be combined or distributed as desired in various embodiments. In addition, the functionality may be embodied in whole or in part in firmware or hardware equivalents, such as integrated circuits, application-specific integrated circuits (ASICs), field programmable gate arrays (FPGA), and the like. Particular data structures may be used to more effectively implement one or more aspects of the disclosure, and such data structures are contemplated to be within the scope of computer executable instructions and computer-usable data described herein.

One or more aspects described herein may be embodied as a method, an apparatus, or as one or more computer-readable media storing computer-executable instructions. Accordingly, those aspects may take the form of an entirely hardware embodiment, an entirely software embodiment, an entirely firmware embodiment, or an embodiment combining software, hardware, and firmware aspects in any combination. In addition, various signals representing data or events as described herein may be transferred between a source and a destination in the form of light or electromagnetic waves traveling through signal-conducting media such as metal wires, optical fibers, or wireless transmission media (e.g., air or space). The one or more computer-readable media may be and/or include one or more non-transitory computer-readable media.

As described herein, the various methods and acts may be operative across one or more computing servers and one or more networks. The functionality may be distributed in any manner, or may be located in a single computing device (e.g., a server, a client computer, and the like). For example, in alternative embodiments, one or more of the computing platforms discussed above may be combined into a single computing platform, and the various functions of each computing platform may be performed by the single computing platform. In such arrangements, any and/or all of the above-discussed communications between computing platforms may correspond to data being accessed, moved, modified, updated, and/or otherwise used by the single computing platform. Additionally or alternatively, one or more of the computing platforms discussed above may be implemented in one or more virtual machines that are provided by one or more physical computing devices. In such arrangements, the various functions of each computing platform may be performed by the one or more virtual machines, and any and/or all of the above-discussed communications between computing platforms may correspond to data being accessed, moved, modified, updated, and/or otherwise used by the one or more virtual machines.

Aspects of the disclosure have been described in terms of illustrative embodiments thereof. One or more of the steps depicted in the illustrative figures may be performed in other than the recited order, and one or more depicted steps may be optional in accordance with aspects of the disclosure.

## Claims

1. A traceable vial (100) comprising:
a vial (110) configured to hold contents therein; and
a tracking assembly (120, 200) fitted to an exterior portion of the vial and housing electronic components configured to transmit a signal indicating a status of the vial;
wherein the tracking assembly (120, 200) includes an antenna (220) configured to transmit the signal and a battery (230) configured to power the antenna (220), **characterized in that**:
the tracking assembly (120, 200) includes a temperature sensor (260) and the antenna (220) is configured to transmit a signal indicating a temperature reading by the temperature sensor (260), and
the tracking assembly (120, 200) is structured and designed to fit around a lower base portion of the vial (110).

2. The traceable vial (100) of claim 1, wherein the tracking assembly (120, 200) encircles a base of the vial (110).

3. The traceable vial (100) of claim 1, wherein the electronic components housed in the tracking assembly are configured to transmit the signal using a narrowband internet of things (NB-IOT) network.

4. The traceable vial (100) of claim 1, wherein the electronic components housed in the tracking assembly (120, 200) are configured to transmit the signal indicating at least one of: a vial identification, a location identification, a battery voltage, a coverage level, a radio frequency band, or a measured temperature.

5. The traceable vial (100) of claim 1, wherein the electronic components housed in the tracking assembly (120, 200) are configured to transmit the signal to a base transceiver station within a network, and wherein lookup information associated with nearest base transceiver station indicates a location range of the traceable vial (100).

6. The traceable vial (100) of claim 5, wherein the electronic components transmitting the signal to the base transceiver station are configured to provide location mapping information including at least one of: a location name, a longitude, a latitude, or a location timestamp.

7. The traceable vial (100) according to claim 1, wherein the antenna (220) is a NB-IoT antenna and the tracking assembly further comprises a Wi-Fi antenna,
wherein the Wi-Fi antenna is configured to detect the presence of a local Wi-Fi signal and capture an identifier for that Wi-Fi signal, and
the NB-IoT antenna is configured to transmit the identifier for the Wi-Fi signal to a base transceiver station for determining a location of the traceable vial (100).

8. The traceable vial (100) according to claim 1 wherein the antenna (220) is configured to transmit measurement information containing the temperature reading to a mobile user device in a threshold vicinity of the traceable medical vial (100), the mobile user device being configured to transmit a corresponding signal based on a location of the mobile user device to an administrator computing device.

9. The traceable vial (100) of claim 1, wherein the electronic components housed in the tracking assembly (120, 200) are configured to transmit one of two messages in an alternating manner for a set update period, and wherein a first message includes a device identification information, and a second message includes telemetry information associated with the traceable vial (100), wherein the telemetry information associated with the traceable vial (100) includes the temperature reading.

10. The traceable vial (100) of any preceding claim wherein the electronic components housed in the tracking assembly (120, 200) are configured to transmit signals at a frequency that varies based on a sensed movement of the traceable vial (100).

11. The traceable vial (100) of claim 1, wherein the tracking assembly (120, 200) is configured to deactivate upon receiving deactivation instructions from an administrator computing device or an end user computing device.

12. The traceable vial (100) of claim 1, wherein the vial (110) is composed of medical grade glass.

13. A traceable vial system comprising:
at least one traceable vial (100) including:
a vial (110); and
a tracking assembly (120, 200) fitted to an exterior portion of the vial and housing electronic components configured to transmit a signal; and
a receiver configured to receive the signal from the at least one traceable vial (100) and to detect one or more metrics of the at least one traceable vial (100) based on the signal;
wherein the tracking assembly includes an antenna (220) configured to transmit the signal and a battery (230) configured to power the antenna (220), **characterized in that**:
the tracking assembly (120, 200) includes a temperature sensor (260) and the antenna (220) is configured to transmit a signal indicating a temperature reading by the temperature sensor (260), and
the tracking assembly (120, 200) is structured and designed to fit around a lower base portion of the vial (110).

14. The traceable vial system of claim 13, wherein the receiver is housed in a mobile user device, and wherein the mobile user device executes an application that processes the signal received from the traceable vial (100) and optionally transmits a second signal to an administrator computing device, wherein the second signal includes location information identifying a location of the mobile user device when the signal was received from the traceable vial (100).

15. The traceable vial system of claim 13, wherein the signal indicates a location information and a timestamp of when the signal was received.

## Patentansprüche

1. Verfolgbare Phiole (100), umfassend:
eine Phiole (110), die dafür ausgebildet ist, Inhalte in sich zu halten; und
eine Nachverfolgungseinrichtung (120, 200), die an einen Außenabschnitt der Phiole angepasst ist und in der Elektronikkomponenten untergebracht sind, die dafür ausgebildet sind, ein Signal zu senden, das einen Status der Phiole angibt;
wobei die Nachverfolgungseinrichtung (120, 200) eine Antenne (220), die dafür ausgebildet ist, das Signal zu senden, und eine Batterie (230), die dafür ausgebildet ist, die Antenne (220) zu speisen, einschließt, **dadurch gekennzeichnet, dass**:
die Nachverfolgungseinrichtung (120, 200) einen Temperatursensor (260) einschließt und die Antenne (220) dafür ausgebildet ist, ein Signal zu senden, das einen Temperaturmesswert von dem Temperatursensor (260) angibt, und
die Nachverfolgungseinrichtung (120, 200) so konstruiert und gestaltet ist, dass sie um einen unteren Bodenabschnitt der Phiole (110) passt.

2. Verfolgbare Phiole (100) nach Anspruch 1, wobei die Nachverfolgungseinrichtung (120, 200) einen Boden der Phiole (110) umschließt.

3. Verfolgbare Phiole (100) nach Anspruch 1, wobei die in der Nachverfolgungseinrichtung untergebrachten Elektronikkomponenten dafür ausgebildet sind, das Signal unter Verwendung eines Schmalband-Internet-der-Dinge-(NB-IOT-)Netzwerkes zu senden.

4. Verfolgbare Phiole (100) nach Anspruch 1, wobei die in der Nachverfolgungseinrichtung (120, 200) untergebrachten Elektronikkomponenten dafür ausgebildet sind, das Signal, das mindestens eines von Folgendem angibt, zu senden: eine Phiolenidentifikation, eine Standortidentifikation, eine Batteriespannung, einen Abdeckungsgrad, ein Hochfrequenzband oder eine gemessene Temperatur.

5. Verfolgbare Phiole (100) nach Anspruch 1, wobei die in der Nachverfolgungseinrichtung (120, 200) untergebrachten Elektronikkomponenten dafür ausgebildet sind, das Signal an eine Basis-Sende-/Empfangsstation innerhalb eines Netzwerkes zu senden, und wobei Verweisinformationen in Verbindung mit der nächstgelegenen Basis-Sende-/Empfangsstation einen Standortbereich der verfolgbaren Phiole (100) angeben.

6. Verfolgbare Phiole (100) nach Anspruch 5, wobei die Elektronikkomponenten, die das Signal an die Basis-Sende-/Empfangsstation senden, dafür ausgebildet sind, Standortkartierungsinformationen bereitzustellen, die mindestens eines von Folgendem einschließen: einen Standortnamen, einen Längengrad, einen Breitengrad oder einen Standortzeitstempel.

7. Verfolgbare Phiole (100) nach Anspruch 1, wobei die Antenne (220) eine NB-IoT-Antenne ist und die Nachverfolgungseinrichtung ferner eine Wi-Fi-Antenne umfasst,
wobei die Wi-Fi-Antenne dafür ausgebildet ist, die Gegenwart eines lokalen Wi-Fi-Signals zu erfassen und eine Identifikation für dieses Wi-Fi-Signal einzufangen, und
die NB-IoT-Antenne dafür ausgebildet ist, die Identifikation für das Wi-Fi-Signal an eine Basis-Sende-/Empfangsstation zum Bestimmen eines Standorts der verfolgbaren Phiole (100) zu senden.

8. Verfolgbare Phiole (100) nach Anspruch 1, wobei die Antenne (220) dafür ausgebildet ist, Messungsinformationen, die den Temperaturmesswert enthalten, an eine mobile Benutzervorrichtung in einem Schwellenwertumfeld der verfolgbaren medizinischen Phiole (100) zu senden, wobei die mobile Benutzervorrichtung dafür ausgebildet ist, ein entsprechendes Signal basierend auf einem Standort der mobilen Benutzervorrichtung an eine Administratorrechenvorrichtung zu senden.

9. Verfolgbare Phiole (100) nach Anspruch 1, wobei die in der Nachverfolgungseinrichtung (120, 200) untergebrachten Elektronikkomponenten dafür ausgebildet sind, eine von zwei Nachrichten abwechselnd für einen festgelegten Aktualisierungszeitraum zu senden, und wobei eine erste Nachricht eine Vorrichtungsidentifikationsinformation einschließt und eine zweite Nachricht Telemetrieinformationen in Verbindung mit der verfolgbaren Phiole (100) einschließt, wobei die Telemetrieinformationen in Verbindung mit der verfolgbaren Phiole (100) den Temperaturmesswert einschließen.

10. Verfolgbare Phiole (100) nach einem vorstehenden Anspruch, wobei die in der Nachverfolgungseinrichtung (120, 200) untergebrachten Elektronikkomponenten dafür ausgebildet sind, Signale mit einer Frequenz, die basierend auf einer erfassten Bewegung der verfolgbaren Phiole (100) variiert, zu senden.

11. Verfolgbare Phiole (100) nach Anspruch 1, wobei die Nachverfolgungseinrichtung (120, 200) dafür ausgebildet ist, sich beim Empfangen von Deaktivierungsanweisungen von einer Administratorrechenvorrichtung oder einer Endbenutzerrechenvorrichtung zu deaktivieren.

12. Verfolgbare Phiole (100) nach Anspruch 1, wobei die Phiole (110) aus medizinischem Glas besteht.

13. System mit einer verfolgbaren Phiole, umfassend:
mindestens eine verfolgbare Phiole (100), die Folgendes einschließt:
eine Phiole (110); und
eine Nachverfolgungseinrichtung (120, 200), die an einen Außenabschnitt der Phiole angepasst ist und in der Elektronikkomponenten untergebracht sind, die dafür ausgebildet sind, ein Signal zu senden, und
einen Empfänger, der dafür ausgebildet ist, das Signal von der mindestens einen verfolgbaren Phiole (100) zu empfangen und eine oder mehrere Metrik(en) der mindestens einen verfolgbaren Phiole (100) basierend auf dem Signal nachzuweisen;
wobei die Nachverfolgungseinrichtung eine Antenne (220), die dafür ausgebildet ist, das Signal zu senden, und eine Batterie (230), die dafür ausgebildet ist, die Antenne (220) zu speisen, einschließt, **dadurch gekennzeichnet, dass**:
die Nachverfolgungseinrichtung (120, 200) einen Temperatursensor (260) einschließt und die Antenne (220) dafür ausgebildet ist, ein Signal zu senden, das einen Temperaturmesswert von dem Temperatursensor (260) angibt, und
die Nachverfolgungseinrichtung (120, 200) so konstruiert und gestaltet ist, dass sie um einen unteren Bodenabschnitt der Phiole (110) passt.

14. System mit einer verfolgbaren Phiole nach Anspruch 13, wobei der Empfänger in einer mobilen Benutzervorrichtung untergebracht ist und wobei die mobile Benutzervorrichtung eine Anwendung ausführt, die das von der verfolgbaren Phiole (100) empfangene Signal verarbeitet und gegebenenfalls ein zweites Signal an eine Administratorrechenvorrichtung sendet, wobei das zweite Signal Standortinformationen einschließt, die einen Standort der mobilen Benutzervorrichtung angeben, sobald das Signal von der verfolgbaren Phiole (100) empfangen wurde.

15. System mit einer verfolgbaren Phiole nach Anspruch 13, wobei das Signal eine Standortinformation und einen Zeitstempel, wann das Signal empfangen wurde, angibt.

## Revendications

1. Flacon traçable (100) comprenant :
un flacon (110) configuré pour maintenir un contenu à l'intérieur de celui-ci ; et
un ensemble de suivi (120, 200) ajusté sur une partie extérieure du flacon et logeant des composants électroniques configurés pour émettre un signal indiquant un état du flacon ;
dans lequel l'ensemble de suivi (120, 200) comprend une antenne (220) configurée pour émettre le signal et une batterie (230) configurée pour alimenter l'antenne (220), **caractérisé en ce que** :
l'ensemble de suivi (120, 200) comprend un capteur de température (260) et l'antenne (220) est configurée pour émettre un signal indiquant une lecture de température par le capteur de température (260), et
l'ensemble de suivi (120, 200) est structuré et conçu pour s'ajuster autour d'une partie de base inférieure du flacon (110).

2. Flacon traçable (100) selon la revendication 1, dans lequel l'ensemble de suivi (120, 200) entoure une base du flacon (110).

3. Flacon traçable (100) selon la revendication 1, dans lequel les composants électroniques logés dans l'ensemble de suivi sont configurés pour émettre le signal en utilisant un réseau Internet des objets à bande étroite (NB-IOT).

4. Flacon traçable (100) selon la revendication 1, dans lequel les composants électroniques logés dans l'ensemble de suivi (120, 200) sont configurés pour émettre le signal indiquant au moins un parmi : une identification de flacon, une identification d'emplacement, une tension de batterie, un niveau de couverture, une bande de fréquence radio ou une température mesurée.

5. Flacon traçable (100) selon la revendication 1, dans lequel les composants électroniques logés dans l'ensemble de suivi (120, 200) sont configurés pour émettre le signal à une station émettrice-réceptrice de base au sein d'un réseau, et dans lequel les informations de correspondance associées à la station émettrice-réceptrice de base la plus proche indiquent une plage d'emplacement du flacon traçable (100).

6. Flacon traçable (100) selon la revendication 5, dans lequel les composants électroniques émettant le signal à la station émettrice-réceptrice de base sont configurés pour fournir des informations de cartographie d'emplacement comprenant au moins un parmi : un nom d'emplacement, une longitude, une latitude ou un horodatage d'emplacement.

7. Flacon traçable (100) selon la revendication 1, dans lequel l'antenne (220) est une antenne NB-IoT et l'ensemble de suivi comprend en outre une antenne Wi-Fi,
dans lequel l'antenne Wi-Fi est configurée pour détecter la présence d'un signal Wi-Fi local et capturer un identifiant pour ce signal Wi-Fi, et
l'antenne NB-IoT est configurée pour émettre l'identifiant du signal Wi-Fi à une station émettrice-réceptrice de base afin de déterminer un emplacement du flacon traçable (100).

8. Flacon traçable (100) selon la revendication 1 dans lequel l'antenne (220) est configurée pour émettre des informations de mesure contenant la lecture de température à un dispositif utilisateur mobile dans un voisinage seuil du flacon médical traçable (100), le dispositif utilisateur mobile étant configuré pour émettre un signal correspondant basé sur un emplacement du dispositif utilisateur mobile à un dispositif informatique administrateur.

9. Flacon traçable (100) selon la revendication 1, dans lequel les composants électroniques logés dans l'ensemble de suivi (120, 200) sont configurés pour émettre un parmi deux messages de manière alternée pendant une période de mise à jour définie, et dans lequel un premier message comprend des informations d'identification de dispositif, et un second message comprend des informations de télémétrie associées au flacon traçable (100), dans lequel les informations de télémétrie associées au flacon traçable (100) comprennent la lecture de température.

10. Flacon traçable (100) selon l'une quelconque des revendications précédentes, dans lequel les composants électroniques logés dans l'ensemble de suivi (120, 200) sont configurés pour émettre des signaux à une fréquence qui varie sur la base d'un mouvement détecté du flacon traçable (100).

11. Flacon traçable (100) selon la revendication 1, dans lequel l'ensemble de suivi (120, 200) est configuré pour se désactiver lors de la réception d'instructions de désactivation provenant d'un dispositif informatique administrateur ou d'un dispositif informatique utilisateur final.

12. Flacon traçable (100) selon la revendication 1, dans lequel le flacon (110) est composé de verre de qualité médicale.

13. Système de flacons traçables comprenant :
au moins un flacon traçable (100) comprenant :
un flacon (110) ; et
un ensemble de suivi (120, 200) ajusté sur une partie extérieure du flacon et logeant des composants électroniques configurés pour émettre un signal ; et
un récepteur configuré pour recevoir le signal provenant de l'au moins un flacon traçable (100) et pour détecter une ou plusieurs métriques de l'au moins un flacon traçable (100) sur la base du signal ;
dans lequel l'ensemble de suivi comprend une antenne (220) configurée pour émettre le signal et une batterie (230) configurée pour alimenter l'antenne (220), **caractérisé en ce que** :
l'ensemble de suivi (120, 200) comprend un capteur de température (260) et l'antenne (220) est configurée pour émettre un signal indiquant une lecture de température par le capteur de température (260), et
l'ensemble de suivi (120, 200) est structuré et conçu pour s'ajuster autour d'une partie de base inférieure du flacon (110).

14. Système de flacons traçables selon la revendication 13, dans lequel le récepteur est logé dans un dispositif utilisateur mobile, et dans lequel le dispositif utilisateur mobile exécute une application qui traite le signal reçu du flacon traçable (100) et émet éventuellement un second signal à un dispositif informatique administrateur, dans lequel le second signal comprend des informations d'emplacement identifiant un emplacement du dispositif utilisateur mobile lorsque le signal a été reçu du flacon traçable (100).

15. Système de flacons traçables selon la revendication 13, dans lequel le signal indique des informations d'emplacement et un horodatage du moment où le signal a été reçu.
